(19) Europäisches Patentamt
European Patent Office
Office européen des brevets

(11) Publication number: **0 399 133 B1**

(12) **EUROPEAN PATENT SPECIFICATION**

(45) Date of publication of patent specification: **09.03.94**

(51) Int. Cl.5: **C07C 229/24**, C11D 3/33

(21) Application number: **89870068.7**

(22) Date of filing: **23.05.89**

The file contains technical information submitted after the application was filed and not included in this specification

(54) **Detergent and cleaning compositions containing chelating agents.**

(43) Date of publication of application:
**28.11.90 Bulletin 90/48**

(45) Publication of the grant of the patent:
**09.03.94 Bulletin 94/10**

(84) Designated Contracting States:
**AT BE CH DE ES FR GB GR IT LI LU NL SE**

(56) References cited:
**EP-A- 0 262 112**
**EP-A- 0 317 542**
**FR-A- 2 177 800**
**FR-A- 2 285 869**
**US-A- 4 416 792**

**JOURNAL OF THE CHEMICAL SOCIETY - DALTON TRANSACTIONS, no. 13, 1976, pages 1207-1212; M. B. JONES et al, "Nickel Complexes of N-Substituted Iminodiacetates in Aqueous Solution: Co-ordination by the Hydroxyl Group of Hydroxyalkyl Substituents"**

(73) Proprietor: **THE PROCTER & GAMBLE COMPANY**
**One Procter & Gamble Plaza**
**Cincinnati Ohio 45202(US)**

(72) Inventor: **Vos, Eddy**
**Koetsiersweg 15**
**B-3202 Linden(BE)**
Inventor: **Corn, Ian**
**Klauwaartslaan 93**
**B-1820 Strombeek-Bever(BE)**
Inventor: **Konig, Axel**
**Erasmuslaan 19**
**B-1810 Wemmel(BE)**

(74) Representative: **Canonici, Jean-Jacques et al**
**Procter & Gamble European Technical Center N.V.**
**Temselaan 100**
**B-1853 Strombeek-Bever (BE)**

EP 0 399 133 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to detergent and cleaning compositions in particular hard-surface cleaning compositions, containing chelating agents derived from iminodiacetic acid.

### Background of the Invention

Several compounds of the iminodiacetic acid type have been synthesised and investigated for the purpose of metal sequestering properties.

For example, methyl iminodiacetic acid, whose synthesis is described in Chemical Abstracts 104(6)-45062d, Acetamido Imino Diacetic acid, described in Biochemistry 5, p 467 (1966), Glyceryl Imino Diacetic acid, described in Chem. ZUESTI 34(1) p. 93-103 (1980) MAYER, RIECANSKA, et al publication of 26 March 1979.

The compounds 2-hydroxyethyl diacetic acid, diethyleneglycol diacetic acid and 1-hydroxypropyl imino diacetic acid have been disclosed in Japanese Laid-Open Application 59/70652;

Other iminodiacetic derivatives such as 2-hydroxypropylimino diacetic acid, and di-hydroxypropyl imino diacetic acid have been disclosed in DE-OS 23 14 449, and DE-OS 25 42 708;

Several of the above compounds have been described as chelating agents in detergent compositions. EPA 262 112 describes the use of diethyleneglycol diacetic acid as builder in detergent compositions, including hard-surface cleaning compositions.

EP-A-317 542 discloses the combination of specific imino acetic derivatives such as Glyceryl Imino Diacetic Acid and Acetamido Imino Diacetic Acid with certain solvents, in hard-surface cleaning compositions.

Iminodiacetic acid derivatives have now been found to be particularly performing as chelating agents in detergent/cleaning compositions

### Summary of the Invention

The present invention relates to detergent compositions and in particular hard-surface cleaning compositions, containing chelating agents derived from iminodiacetic acid, having the formula

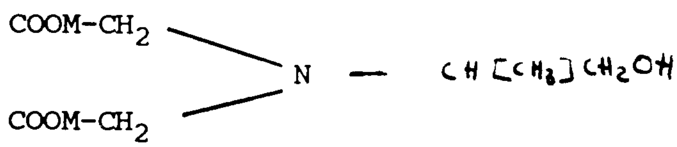

wherein M is either hydrogen or a salt-forming cation.

### Detailed Description of the Invention

#### The chelating agents

The chelating agents of the present invention are of the formula :

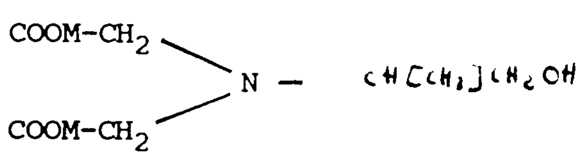

wherein M is either hydrogen or a salt-forming cation, preferably Na, K, $NH_4^+$ or substituted ammonium cations containing from 1 to 4 short-chain alkyl or hydroxy alkyl groups each of which containing from 1 to 3 carbon atoms.

The following chelating agent is thus encompassed by the present invention (M being hydrogen) :

Iso Hydroxy Propyl Imino Diacetic Acid (IHYPIDA), having the following structure :

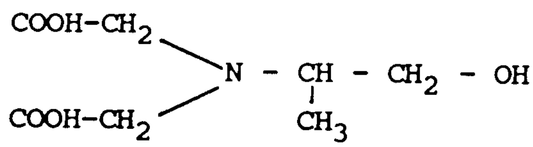

This compound is prepared by the following reaction :

$$2ClCH_2COONa + H_2N\ CH(CH_3)CH_2OH + Na_2CO_3$$

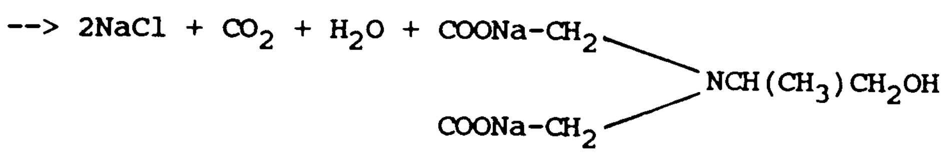

Detailed method of preparation of the chelating agents (sodium salt)

About 0.86mole of - aminoisopropanol (for IHYPIDA) and about 1.7mole sodium chloroacetate are slurried in 500mls water in a 1 litre conical fitted with a reflux condenser. 0.86mole sodium carbonate is added carefully and the mixture heated to at least 50 °C for over 2 hours then at least 95 °C for over 3 hours. On cooling, the solution is acidified to pH 2 with conc. HCL and evaporated under reduced pressure. The solid is hot ethanol extracted and evaporated to dryness again. A slurry of the solid in water is resaponified at pH 11 (conc. sodium hydroxide) for over 1 hour at least 60 °C then evaporated to dryness.

Detergent/cleaning compositions

The iminodiacetic acid derivatives herein are useful as chelating agents in detergent/cleaning compositions which encompass laundry detergent compositions as well as hard-surface cleaning compositions. The application as chelating agents in hard-surface cleaning compositions is especially preferred.

Detergent compositions according to the present invention contain from 0.01% to 95% of an organic synthetic surfactant and from 0.05% to 95% of a chelating agent herein.

Organic synthetic surfactants useful herein include well-known synthetic anionic, nonionic, cationic, amphoteric and zwitterionic surfactants and mixtures thereof. Typical of these are the alkyl sulfates, alkyl benzene sulfates and sulfonates, paraffin sulfonates, olefin sulfonates, alkoxylated (especially ethoxylated) alcohols, alkyl phenols, and sulfates, amine oxides and sulfonates of fatty acids and of fatty acid esters, which are well-known in the detergency art. In general, such detersive surfactants contain an alkyl group in the $C_{10}$-$C_{18}$ range; the anionic detersive surfactants can be used in the form of their sodium, potassium or ammonium salts. The nonionic generally contain from 3 to 17 ethylene oxide groups per mole of hydrophobic moiety. Cationic surfactants will generally be represented by quaternary ammonium compounds such as ditallow dimethyl ammonium chloride, and will be preferably used in combination with nonionic surfactants.

Especially preferred in the compositions of the present invention are : $C_{12}$-$C_{16}$ alkyl benzene sulfonates, $C_{12}$-$C_{18}$ paraffin-sulfonates and the ethoxylated alcohols of the formula $RO(CH_2CH_2O)_n$, with R being a $C_{12}$-$C_{15}$ alkyl chain and n being a number from 6 to 10, and the ethoxylated alcohol sulfates of formula $RO\text{-}(CH_2CH_2O)_n\text{-}SO_3M$, with R being a $C_{12}$-$C_{18}$ alkyl chain on a number from 2 to 8, and M is H or an alkalimetal ion.

Laundry detergent compositions according to the present invention typically contain from 1% to 40% preferably 5% to 30% of organic synthetic surfactant, and from 1% to 40% preferably 5% to 30% of a chelating agent herein.

Said laundry detergent compositions may be formulated into granules, liquids, solid tablets or bar forms; said compositions may contain the various adjuncts which are known to the art for laundry detergent compositions. Non-limiting examples of such adjuncts are :
Additional detergency builders such as polyphosphates (e.g., potassium pyrophosphate), nitrilotriacetates

(e.g., $Na_3NTA$), sodium ethylenediaminetetraacetate, sodium ethylenetriaminepentaacetate, sodium citrate, sodium carbonate, sodium metasilicate and zeolites, e.g., zeolites having a cation exchange capacity (measured as $CaCO_3$) of 200 mg or greater per gram of zeolite;
Enzymes such as proteases and amylases;
Bleaches such as sodium perborate, diperoxydodecanedioic acid, sodium dicholorisocyanurate and m-cholorperoxybenzoic acid;
Soil suspending agents such as sodium carboxymethyl cellulose;
Bleach activators for use with sodium perborate, such as tetraacetyl ethylene diamine and sodium nonanoyloxybenzene sulfonate;
Bleach stabilizers such as sodium diethylenetriamine-pentamethylenephosphonate and sodium diethylenetriaminipentaacetate;
Hydrotropes such as sodium toluene sulfonate, sodium cumene sulfonate and potassium xylene sulfonate;
Fabric softening ingredients such as smectite clay, ditallowdimethylamine, ditallowacetamide, ditallowbenzamide, ditallowdimethylammonium chloride.

Hard-surface cleaning compositions according to the present invention typically contain from 0.01% to 45% of organic synthetic surfactant, and from 0.2% to 20%, preferably 0.2% to 10% of a chelating agent herein.

It is preferred that the hard-surface cleaning compositions herein also contain from 1% to 20% of an organic solvent having a boiling point above 90 ° C;

It has been found indeed that such solvents are particularly suitable for use in combination with the present chelating agents, the combination giving unexpected soil-release benefits.

Such benefits are particularly noticeable in terms of calcium soap-soil removal from surfaces such as bathtub surfaces.

It has been found that, in order to obtain such an effect in hard-surface cleaning compositions not of the spray type, the weight ratio of said organic solvent to chelating agent should be in the range from 2/3 to 2/1, preferably 1/1 to 2/1.

In the non-spray type compositions, it is preferable not to include $C_1$-$C_3$ aliphatic alcohols like isopropanol (B.P. 82 ° C) which do not constitute such an effective combination with the present chelating agents.

In spray cleaners however, $C_{1-3}$ aliphatic alcohols such as ethanol and isopropanol are preferably used, in combination with a solvent having a boiling point above 90 ° C.

Representatives of organic solvents with boiling point above 90 ° C which are effective in the present context are : $C_6$-$C_9$ alkyl aromatic solvents, especially the $C_6$-$C_9$ alkyl benzenes, alpha-olefins, like 1-decene or 1-dodecene, benzyl alcohol, n-hexanol, phthalic acid esters.

A type of these solvent especially suitable for the compositions herein comprises diols having from 6 to 16, preferably 8 to 12, carbon atoms in their molecular structure. Preferred diol solvents have a solubility in water of from about 0.1 to about 20g/100g of water at 20 ° C. The most preferred diol solvents are 2,2,4-trimethyl-1,3-pentanediol, and 2-ethyl-1,3-hexanediol.

Glycol ethers are another class of particularly preferred solvents.

In this category, are : water-soluble CARBITOL $^R$ solvents or water-soluble CELLOSOLVE $^R$ solvents. Water-soluble CARBITOL $^R$ solvents are compounds of the 2-(2-alkoxyethoxy)ethanol class wherein the alkoxy group is derived from ethyl, propyl, butyl pentyl hexyl; a preferred water-soluble carbitol is 2-(2-butoxyethoxy)ethanol also known as butyl carbitol. Preferred are also hexyl carbitol and 2-methyl pentyl carbitol. Water-soluble CELLOSOLVE $^R$ solvents are compounds of the 2-alkoxyethoxy ethanol class, wherein the alkoxy group is preferably butyl or hexyl.

Still in the glycol ether catergory, certain propylene-glycol derivatives have been found to be particularly efficient in the present context; these species include 1-n butoxypropane-2-ol, and 1(2-n-butoxy-1 methylethoxy)propane-2-ol butoxypropoxypropanol), with the latter being especially preferred.

Mixtures of the above solvents can also be used, like Butyl carbitol and/or Benzyl alcohol together with diols and/or glycol ethers.

The organic solvent having a boiling point above 90 ° C is preferably present at levels of from 1% to 10% of the total composition.

In spray-cleaners, preferred are mixtures of 1-n butoxypropane-2-ol with ethanol or isopropanol, in a ratio of from 1 to 20 to 1 to 2.

Hard-surfaces cleaning compositions according to the present invention may contain additional (optional) ingredients.

Such ingredients can be represented by conventional detergency builders, which may be used in addition to the chelating agent herein; compounds classifiable and well-known in the art as detergent

builders include the nitrilotriacetates (NTA), polycarboxylates, citrates, water-soluble phosphates such as tri-polyphosphate and sodium ortho- and pyro-phosphates, silicates, ethylene diamine tetraacetate (EDTA), amino-polyphosphonates (DEQUEST), phosphates and mixtures thereof.

Highly desirable ingredients for use herein are represented by conventional detergent hydrotropes. Examples of suitable hydrotropes are urea, monoethanolamine, diethanolamine, triethanolamine and the sodium potassium, ammonium and alkanol ammonium salts of xylene-, toluene-, ethylebenzene- and isopropyl-benzene (cumene) sulfonates.

The hard-surface cleaning compositions of the invention may also contain an abrasive material.

The abrasives suitable herein are selected from water-insoluble, non-gritty materials well-known in the literature for their relatively mild abrasive properties. It is highly preferred that the abrasives used herein not be undesirable "scratchy". Abrasive materials having a Mohs hardness in the range of about 7, or below, are typically used; abrasives having a Mohs hardness of 3, or below, can be used to avoid scratches on aluminium or stainless steel finishes. Suitable abrasives herein include inorganic materials, especially such materials as calcium carbonate and diatomaceous earth, as well as materials such as Fuller's earth, magnesium carbonate, China clay, actapulgite, calcium hydroxyapatite, calcium orthophosphate, dolomite and the like. The aforesaid inorganic materials can be qualified as "strong abrasives". Organic abrasives such as urea-formaldehyde, methyl methacrylate melamine-formaldehyde resins, polyethylene spheres and polyvinylchloride can be advantageously used in order to avoid scratching on certain surfaces, especially plastic surfaces.

Typically, abrasives have a particle size range of 10-1000 microns and are used at concentrations of 5% to 30% in the compositions. Thickeners are frequently added to suspend the abrasives.

Thickeners will preferably be included in the compositions of the inventions, mainly in order to suspend the abrasive; high levels of thickener are detrimental to the performance because they are difficult to rinse from the cleaned surfaces. Accordingly, the level will be kept under 2%, preferably from 0.2% to 1.5%.Common thickeners such as the polyacrylates, xanthan gums, carboxymethyl celluloses, swellable smectite clays, and the like, can be used herein.

Soaps can be included in the compositions herein, the soaps prepared from coconut oil fatty acids being preferred.

Optional components are also represented by ingredients typically used in commercial products to provide aesthetic or additional product performance benefits. Typical ingredients include perfumes, dyes, optical brighteners, soil suspending agents, detersive enzymes, gel-control agents, thickeners, freeze-thaw stabilizers, bactericides, preservatives, and the like,

The hard-surface cleaning compositions herein will advantageously be executed in the form of an aqueous liquid compositions, including concentrates, containing as essential ingredients a surface-active agent, and a solvent/chelating agent binary mixture such as described above.

Liquid executions at normal dilution usually contain 1-6% surfactant and 8-20% solvent/chelating agent binary mixture.

Concentrated liquid executions usually contain 6-10% surfactant and 16-24% solvent/chelating agent binary mixture.

The present invention also encompasses window-cleaners or all-purpose cleaners designed to be applied via sprays, wherein the surfactants are typically used at levels of from 0.01 to 2% and chelants are used at levels of from 0.2% to 3%.

Alternatively, the compositions herein will be in the form of a creamy scouring cleanser, containing an abrasive material, surface-active agent, and the solvent/chelating agent binary mixture of the invention.

In above executions, the pH of the compositions will be neutral or in the alkaline range, generally in the range of pH 5-11.

The following examples are given by way of illustrating the compositions herein, but are not intended to be limiting of the scope of the invention.

The following abreviations will be used :

| | |
|---|---|
| IHYPIDA | Iso Hydroxy Propyl Diacetic Acid |
| HCnFA | Narrow cut, hardened, coconut fatty acid |
| ETHD | 2-Ethyl-1,3-hexanediol |
| BPP | Butoxy Propoxy Propanol = 1(2-n-butoxy-1-methylethoxy)propane-2-ol |
| BP | Butoxy Propanol = 1-n-butoxy propane-2-ol |
| NaCS | Sodium cumene sulfonate |
| Sokolan$^R$PHC25 | Crosslinked polyacrylate thickener |

The following liquid hard-surface cleaning compositions according to the present invention are prepared

| Ingredients | Ex I | Ex II | Ex III |
|---|---|---|---|
| $C_{11}$-$C_{13}$ n-Alkyl Benzene Sulfonate (Na) | - | - | 1.0 |
| $C_{13-16}$ paraffin sulfonate (Na) | 2.5 | 6.0 | - |
| $C_{12-14}$ alcohol ethoxylate($EO_7$) | - | 2.0 | - |
| $C_{12-14}$ alcohol ethoxylate($EO_3$) | 1.0 | - | - |
| Benzyl alcohol | - | - | - |
| Butyl Carbitol | - | 7.0 | 3.0 |
| BPP | 6.0 | 3.0 | 7.0 |
| IHYPIDA | 4.0 | 10.0 | 10.0 |
| $Na_2CO_3$ | 3.5 | 1.0 | - |
| NaCS | 8.0 | 1.5 | 6.0 |
| water & minors | UP TO 100 | | |

The following creamy scouring composition according to the invention is also prepared :

| | Ex IV |
|---|---|
| $C_{11-13}$n Alkyl Benzene Sulfonate (Na) | 4.0 |
| $C_{13-16}$ paraffin sulfonate (Na) | - |
| $C_{12-14}$ alcohol ethoxylate ($EO_7$) | - |
| HCnFA | 1.5 |
| Benzyl alcohol | - |
| BPP 3.0 | |
| IHYPIDA | 3.0 |
| $Na_2CO_3$ | 3.0 |
| $CaCO_3$ | - |
| Polyvinylchloride | 10.0 |
| Sokolan$^R$PHC25 | 0.4 |
| water & minors | up to 100 |

The following spray-cleaner according to the present invention is also prepared :

| | Ex V |
|---|---|
| $C_{13-16}$ Alkyl Sulfate (Na) | 0.3 |
| $C_{13-16}$ Alkyl Sulfate, Ethoxylated($EO_3$)(Na) | 0.6 |
| Ethanol | 4.0 |
| BP | 4.0 |
| IHYPIDA | 1.2 |
| Perfume, water, and minors | up to 100 |
| pH | 10.4 |

## Claims

1. A detergent composition containing from 0.01% to 95% of an organic synthetic surfactant and from 0.05% to 95% of a chelating agent of the formula :

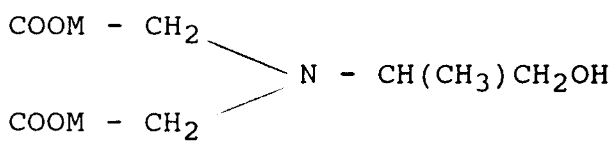

wherein M is either hydrogen or a salt forming cation.

2. A laundry detergent composition according to claim [1] containing from 1% to 40% of an organic synthetic surfactant, and from 1% to 40% of a chelating agent of the formula :

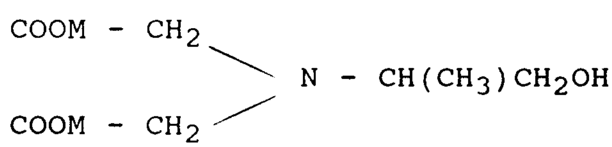

wherein M is either hydrogen or a salt forming cation.

3. A detergent composition according to claim [2] wherein the amount of said organic synthetic surfactant if from 5% to 30% and the amount of chelating agent is from 5% to 30%.

4. A hard-surface cleaning composition according to claim [1] containing from 0.01% to 45% of an organic synthetic surfactant, and from 0.2% to 20% of a chelating agent of the formula :

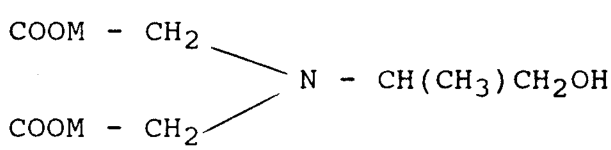

wherein M is either hydrogen or a salt forming cation.

5. A hard-surface cleaning composition according to claim [4] which in addition contains from 1% to 20% of an organic solvent having a boiling point above 90 °C.

6. A hard-surface cleaning composition according to claim [5] which is not of the type suitable as spray-cleaner, and wherein the weight ratio of said organic solvent to chelating agent is from 2/3 to 2/1, preferably 1/1 to 2/1.

7. A hard-surface cleaning composition according to claim [5] wherein the organic solvent is selected from the group of benzyl alcohol, glycol ethers, and diols having 6 to 16 carbon atoms in their molecular structure.

8. A hard-surface cleaning composition according to claim [7] wherein the organic solvent is selected from the group of butoxypropanol, butoxypropoxypropanol, 2-(2-butoxyethoxy-ethanol), benzyl alcohol, 2,2,4,-trimethyl-1,3-pentanediol.

9. A hard-surface cleaning composition according to claim [8] wherein the organic solvent is butoxypropoxypropanol.

10. A hard-surface cleaning composition according to claims [4-9] which in addition contains an abrasive.

11. A hard-surface cleaning composition according to claim [5] which is suitable as spray-cleaner, and which contains, in addition, a solvent selected from the group of ethanol and isopropanol.

12. A hard-surface cleaning composition suitable as spray-cleaner according to claim [11], in which the solvent is represented by a mixture of n-butoxypropanol and ethanol or isopropanol, in a weight ratio of 1:20 to 1:2.

## Patentansprüche

1. Waschmittelzusammensetzung, enthaltend 0,01 % bis 95 % eines organischen synthetischen Tensids und 0,05 % bis 95 % eines Chelatbildners der Formel:

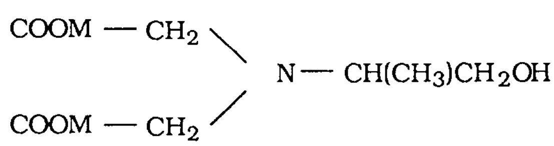

worin M entweder Wasserstoff oder ein salzbildendes Kation ist.

2. Waschmittelzusammensetzung für Wäsche nach Anspruch 1 enthaltend 1 % bis 40 % eines organischen synthetischen Tensids und 1 % bis 40 % eines Chelatbildners der Formel:

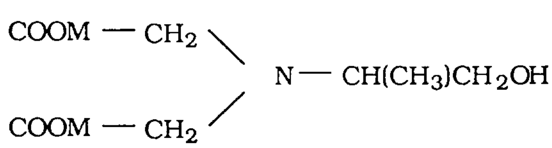

worin M entweder Wasserstoff oder ein salzbildendes Kation ist.

3. Waschmittelzusammensetzung nach Anspruch 2, wobei die Menge des organischen synthetischen Tensids 5 % bis 30 % und die Menge des Chelatbildners 5 % bis 30 % beträgt.

4. Reinigungszusammensetzung für harte Oberflächen nach Anspruch 1, enthaltend 0,01 % bis 45 % eines organischen synthetischen Tensids und 0,2 % bis 20 % eines Chelatbildners der Formel:

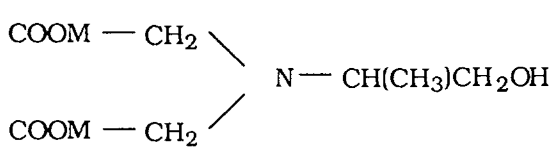

worin M entweder Wasserstoff oder ein salzbildendes Kation ist.

5. Reinigungszusammensetzung für harte Oberflächen nach Anspruch 4, enthaltend weiterhin 1 % bis 20 % eines organischen Lösungsmittels mit einem Siedepunkt oberhalb 90 ° C.

6. Reinigungszusammensetzung für harte Oberflächen nach Anspruch 5, welche nicht dem als Sprühreiniger geeigneten Typ angehört, wobei das Gewichtsverhältnis des organischen Lösungsmittels zu dem Chelatbildner 2/3 bis 2/1, vorzugsweise 1/1 bis 2/1 beträgt.

7. Reinigungszusammensetzung für harte Oberflächen nach Anspruch 5, wobei das organische Lösungsmittel aus der aus Benzylalkohol, Glykolethern und Diolen mit 6 bis 16 Kohlenstoffatomen in deren Molekülstruktur bestehenden Gruppe ausgewählt ist.

8. Reinigungszusammensetzung für harte Oberflächen nach Anspruch 7, wobei das organische Lösungsmittel aus der aus Butoxypropanol, Butoxypropoxypropanol, 2-(2-Butoxyethoxy-ethanol), Benzylalkohol, 2,2,4,Trimethyl-1,3-pentandiol bestehenden Gruppe gewählt ist.

9. Reinigungszusammensetzung für harte Oberflächen nach Anspruch 8, wobei das organische Lösungsmittel Butoxypropoxypropanol ist.

10. Reinigungszusammensetzung für harte Oberflächen nach den Ansprüchen 4-9, enthaltend weiterhin ein Scheuermittel.

11. Reinigungszusammensetzung für harte Oberflächen nach Anspruch 5, welche als Sprühreiniger geeignet ist, enthaltend weiterhin ein aus der Gruppe Ethanol und Isopropanol gewähltes Lösungsmittel.

12. Reinigungszusammensetzung für harte Oberflächen nach Anspruch 11, welche als Sprühreiniger geeignet ist, wobei das Lösungsmittel eine Mischung aus n-Butoxypropanol und Ethanol oder Isopropanol in einem Gewichtsverhältnis von 1:20 bis 1:2 ist.

**Revendications**

1. Composition détergente contenant de 0,01% à 95% d'un tensioactif synthétique organique et de 0,05% à 95% d'un agent chélateur de formule :

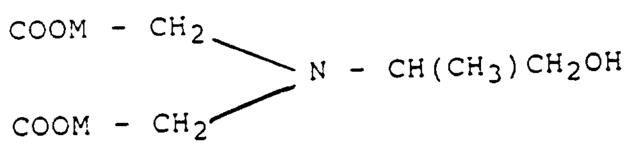

dans laquelle M est un atome d'hydrogène ou un cation formant un sel.

2. Composition détergente pour la lessive selon la revendication 1, contenant de 1% à 40% d'un tensioactif synthétique organique et de 1% à 40% d'un agent chélateur de formule

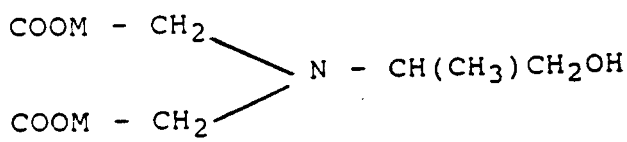

dans laquelle M est un atome d'hydrogène ou un cation formant un sel.

3. Composition détergente selon la revendication 2, dans laquelle la quantité dudit tensioactif synthétique organique est de 5% à 30% et la quantité d'agent chélateur est de 5% à 30%.

**4.** Composition de nettoyage de surfaces dures selon la revendication 1, contenant de 0,01% à 45% d'un tensioactif synthétique organique et de 0,2% à 20% d'un agent chélateur de formule

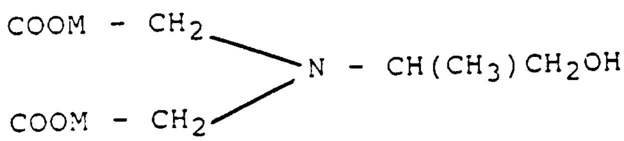

dans laquelle M est un atome d'hydrogène ou un cation formant un sel.

**5.** Composition de nettoyage de surfaces dures selon la revendication 4, qui contient aussi de 1% à 20% d'un solvant organique ayant un point d'ébullition supérieur à 90 ° C.

**6.** Composition de nettoyage de surfaces dures selon la revendication 5, qui ne convient pas comme nettoyant à vaporiser et dans laquelle le rapport pondéral dudit solvant organique à l'agent chélateur est de 2/3 à 2/1, de préférence de 1/1 à 2/1.

**7.** Composition de nettoyage de surfaces dures selon la revendication 5, dans laquelle le solvant organique est choisi dans le groupe constitué de l'alcool benzylique, des éthers de glycol et des diols comportant, dans leur structure moléculaire, de 6 à 16 atomes de carbone.

**8.** Composition de nettoyage de surfaces dures selon la revendication 7, dans laquelle le solvant organique est choisi dans le groupe constitué du butoxypropanol, du butoxypropoxypropanol, du 2-(2-butoxyéthoxy)-éthanol , de l'alcool benzylique, du 2,2,4-triméthyl-1,3-pentanediol.

**9.** Composition de nettoyage de surfaces dures selon la revendication 8, dans laquelle le solvant organique est le butoxypropoxypropanol.

**10.** Composition de nettoyage de surfaces dures selon l'une quelconque des revendications 4-9, qui contient aussi un abrasif.

**11.** Composition de nettoyage de surfaces dures selon la revendication 5, qui convient comme nettoyant à vaporiser et qui contient, en outre, un solvant choisi dans le groupe constitué de l'éthanol et de l'isopropanol.

**12.** Composition de nettoyage de surfaces dures qui convient comme nettoyant à vaporiser selon la revendication 11, dans laquelle le solvant est représenté par un mélange de n-butoxypropanol et d'éthanol ou d'isopropanol, dans un rapport pondéral de 1/20 à 1/2.